(19) Europäisches Patentamt
European Patent Office
Office européen des brevets

(11) **EP 2 489 392 B1**

(12) **EUROPEAN PATENT SPECIFICATION**

(45) Date of publication and mention
of the grant of the patent:
**08.11.2017   Bulletin 2017/45**

(51) Int Cl.:
*A61M 16/12* *(2006.01)*     *B01F 3/02* *(2006.01)*

(21) Application number: **11154899.6**

(22) Date of filing: **17.02.2011**

(54) **Gas blender and method for blending at least two different gases**

Gasmischgerät und Verfahren zum Mischen von mindestens zwei verschiedenen Gasen

Mélangeur de gaz pour mélanger au moins deux gaz différents et procédé de mélange d'au moins deux gaz différents

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**22.08.2012   Bulletin 2012/34**

(73) Proprietor: **Linde AG**
**80331 München (DE)**

(72) Inventor: **Weiszl, Gunther**
**2351 Wiener Neudorf (AT)**

(74) Representative: **Dörries, Hans Ulrich**
**df-mp Dörries Frank-Molnia & Pohlman**
**Patentanwälte Rechtsanwälte PartG mbB**
**Theatinerstrasse 16**
**80333 München (DE)**

(56) References cited:
**WO-A2-2007/064986     US-A- 4 072 148**
**US-A1- 2006 207 594**

## Description

Technical field

The present invention pertains to a gas blender for blending at least two different gases, to a method for blending at least two different gases and to a medical device.

Background of the invention

The administration of blends or mixes of different gases to patients is well known as a treatment for various medical conditions.

For example, obstructive pulmonary diseases may be treated by administering to the respective patient a mixture of helium (He) and oxygen ($O_2$). This type of treatment allows gas to move more swiftly though an airway that is constricted by an asthma attack.

Anesthetic and analgesic effects may be achieved by administering mixtures of nitrous oxide ($N_2O$) and oxygen ($O_2$). This mixture may be administered, for example, during delivery or at the dentist as a pain relief.

When treating patients, maintaining the prescribed blending ratio of the different gases is crucial for the success of the treatment. The required dosing accuracy may range from 1% V/V up to 10% V/V, depending on the clinical efficacy of the mixture at the prescribed concentration set point.

When it comes to the oxygen content of the mixture, it might even be harmful for the patient if the oxygen content drops below the prescribed oxygen content or if it rises above the prescribed oxygen content, depending on the clinical effect of the prescription. In particular, maintaining the correct mixture of oxygen and the respective blending gas or gases is crucial and it is desired to maintain the blending ratio of the gases in a range of $\pm$ 5% V/V, $\pm$ 3% V/V, $\pm$ 2% V/V, more preferred in a range of $\pm$ 1% V/V - depending on the clinical effect of the blending ratio. This accuracy is to be maintained irrespective of the actual gas flow demanded by the patient.

The range of patients to be treated with this specific therapy can be very wide. It may range from a shallow breathing baby to a deep breathing adult. The average maximum flow rates, thus, vary over a wide range of between 10 1/min up to 350 1/min, depending on the size and condition of the respective patient. On the basis of this gas demand of a patient, a device supplying the gas mixture to a patient is required to provide an accurate gas mixture in a flow range of between 0 and 10 1/min up to approximately 0 to 350 1/min - depending on the size and pathological condition of the patient currently connected to the device. However, the dosing accuracy of the concentration of the gas which is supplied to the patient has to be maintained over the entire range of flow rates. It may be appreciated that it is a challenge to provide a device which allows for a high blending accuracy over such a wide range of different and dynamic gas flows. In particular, components for controlling a gas flow are typically inaccurate at lower gas flows because it is difficult to design valves which are accurate at low flows. Furthermore, flow sensors in a closed flow control loop are typically inaccurate at lower flows. The term "lower flow" in this context is intended to refer to a flow which is lower than the flow at the designed set point of the respective device.

The accuracy of dosing is dependent on different parameters in a system. Transitions from laminar to turbulent flows in the system greatly affect the controllability of the system because laminar flows can be easily predicted and controlled whereas turbulent flows tend to have a somewhat chaotic behavior. The operation ranges of valves are typically restricted such that the flows can only be adjusted in a limited range. Flow sensors have given accuracies in given bands only such that flows can be reliably measured within this band only. Accordingly, the flow capacities of dosing systems are restricted to certain values, based, inter alia, on the restrictions of the systems as mentioned before. In particular, devices which are designed to be accurate at lower flows tend to be inaccurate at higher flows as the gas flow typically becomes turbulent and other system parameters do not allow for accurate control of the flow. As soon as the gas flow becomes turbulent in a system due to high demand, the mixing accuracy cannot be maintained. On the other hand, when operating a high flow system at a very low flow rate, the desired accuracy cannot be achieved because the flow sensor may operate outside of its preferred operation range. Accordingly, it is customary to provide two different gas blenders, one for small patients and another one for medium and larger patients in order to accommodate for the different gas flows and for the high accuracy needed for medical applications.

It is also customary to compensate for inaccuracies at lower flows by wasting gas flows into the atmosphere in order to maintain a certain minimum gas flow. As will be appreciated, this is counterproductive in terms of efficient use of gas and/or in terms of avoiding contamination of the atmosphere with certain gases.

Conventional blending systems are constant flow systems in which the flow of two gases is mixed at a constant flow rate. The flow rate is set depending on the demand of the patient. The blended gas is typically supplied to a breathing bag from which the patient takes the blended gas at ambient pressure via a mask. In less sophisticated systems the constant gas flow is simply supplied to the mask where it is administered to the patient while inhaling and is fully wasted during exhaling. In this type of blending systems, the accuracy of the mixing ratio is achieved by keeping a constant gas flow rate at which the behavior of the respective valves and gas conducts is controllable and the gas flows are laminar. In order to maintain the correct mixing ratio, the gas flow is substantially constant for all applications. Accordingly, these systems are not very efficient in terms of gas usage.

WO 2007/064986 A2 discloses a gas blender with an auxiliary mixed gas outlet. The mixing process is carried out through two gas proportional solenoid valves which are controlled in response to a mixing control signal.

US 4,072,148 pertains to a multistage mixing valve for a medical respirator.

Summary of the disclosure

It is an object of the present invention to provide a gas blender and a method for gas blending with increased efficiency over a wide range of gas flows.

The gas blender according to the present invention is intended for blending at least two gases, wherein the gas blender comprises a first gas control valve for supplying a first gas, a second gas control valve for supplying a second gas, a pressure vessel in fluid communication with the first and second gas control valves to receive and store the first and second gases at above atmospheric pressure, and an output gas control valve in fluid communication with an output of the pressure vessel for supplying the blended gas from the pressure vessel.

By means of the provision of the pressure vessel in fluid communication with the first and second gas control valves it becomes possible to provide a gas blender which supplies a blended gas over a wide range of gas flows with a high mixing accuracy and efficient gas usage. In particular, as the two gases from the first and second gas control valves are received in the pressure vessel at above atmospheric pressure, the blended gas is buffered in the pressure vessel in the periodic, non-continuous situation of a patient inhaling and exhaling. Accordingly, the blended gas can be taken from the pressure vessel in a continuous manner at approximately constant pressure.

In order to further increase the efficiency of the gas blender and the efficiency in administration of the blended gas to a patient the output gas control valve preferably is a demand valve, preferably a tilt demand valve, sensing the gas demand of a patient and supplying the blended gas accordingly. By the provision of a demand valve at the output of the pressure vessel, an exact amount of blended gas can be supplied to the patient via the mask without wasting any of the blended gas. As the demand valve is in fluid communication with the output of the pressure vessel the blended gas can always be supplied at an almost constant pressure from the pressure vessel. A sufficient supply of blended gas is always buffered in the pressure vessel such that the blended gas administered to the patient can be supplied to the patient at constant pressure over the whole range of the demanded gas flows as well as over the whole breathing cycle, without wasting gas.

To balance the design considerations with respect to efficiency and building volume, the volume of the pressure vessel may be in the range of 0.01 l to 10 l, preferably 0.1 l to 3 l, more preferred 0.2 l to 0.5 l, most preferred 0.4 l. Depending on the pressure in the pressure vessel these rather small volumes enable on the one hand that the pressure for the patient during one breathing cycle can be maintained substantially at the same level but, on the other hand, changes in the composition of the gases can be effected quickly such that a changed gas composition can be administered to the patient within a few breathing cycles in which the gas inside of the pressure vessel is exchanged by the new mixture.

As the respiratory volume of patients varies over a wide range the maximum flow rate of the first and/or second gas control valve is set to between 5 l/min and 350 l/min, preferably to between 10 l/min to 200 l/min, more preferred to between 140 l/min to 160 l/min, most preferred to 150 l/min in order to accommodate for the gas demand of diverse patients, in particular from shallow breathing pediatrics to deep breathing adults.

Preferably, a controller connected to the first and second gas control valves for controlling the operation of the first and second gas control valves on the basis of a predetermined blending ratio is present. A pressure sensor for sensing the pressure in the pressure vessel may be present, the pressure sensor being connected, preferably via a controller, to the first and second gas control valves for controlling the supply of the first and second gas into the pressure vessel.

The accuracy of the blending process can be further improved by including at least one flow sensor arranged for sensing the flow, preferably the mass flow, of the first and/or the second gas into the pressure vessel, the flow sensor being connected, preferably via a controller, to the first and second gas control valves to control the first and/or second gas control valves on the basis of the gas flow measured by the at least one flow sensor.

The first and/or the second gas control valve may be an electric, hydraulic or pneumatic gas control valve controllable by a controller and/or the first and/or second gas control valve may be a mass flow controller.

In this respect it is to be noted that mass flow controllers typically have their highest accuracy at a specific setpoint which is dependent on the pressure downstream of the mass flow controller. By using the pressure vessel it becomes possible to provide the first and second mass flow controllers with a defined downstream pressure, in particular if a defined pressure range is maintained in the pressure vessel. In other words, because of the defined downstream pressure range provided by the pressure vessel, the first mass flow controller and the second mass flow controller can always work against a defined downstream pressure which can be adjusted in a narrow range, for example between 0.01 and 2 bar, more preferred between 0.2 and 1.5 bar, most preferred between 0.5 and 1.4 bar.

Accordingly, by the introduction of the pressure vessel in fluid communication with the first mass flow controller and the second mass flow controller it becomes possible providing not only a gas blender which is capable of handling a broad range of (periodic) gas flows such that the gas use is efficient, but also to provide for a high accuracy of the mixing ratio in the pressure vessel because the mass flow controllers can work at their optimum setpoint.

Previously, it was almost impossible using a mass flow controller for supplying gases or for mixing gases in medical applications because of the periodic demand of gas in the administration of breathing gases to patients and the accompanying dynamic flow of the gas. In other words, the patient needs a gas supply of between 0 l/min when exhaling, up to a peak inspiratory flow rate (PIFR) of about 350 l/min for a large adult inhaling deeply. Due to this periodic and very dynamic demand, the pressure downstream of the mass flow controllers varied too much for the mass flow controllers to work in a proper range of accuracy. For example, when inhaling, the patient generates a slight under-pressure at the downstream end of the mass flow controller. However, the gas blender of the present disclosure enables using mass flow controllers in medical applications.

In order to keep the pressure in the pressure vessel within a small range of pressure variations, a pressure sensor may be present in the pressure vessel for sensing the pressure of the blended gas in the pressure vessel. In case the pressure drops below a predetermined lower limit in the pressure vessel, the pressure sensor signals this condition to the first and second gas control valves which supply new gas to the pressure vessel at the predetermined mass flow rates. If the pressure in the pressure vessel approaches a predetermined upper limit again, the pressure sensor signals this condition to the first and second gas control valves which consequently seize supplying gas to the pressure vessel.

Preferably, a gas pressure sensor is arranged downstream of the output gas control valve for sensing the gas pressure of the blended gas administered to a patient, the gas pressure sensor preferably being arranged between the output gas control valve and a mask for administering the gas to a patient. A patient interface, preferably in the form of a mask, a nasal mask, a full face mask, a CPCP mask, an anaestetic face mask and/or a mouth piece for administering the blended gas to a patient preferably is in fluid communication with the output side of the output gas control valve.

In order to increase the accuracy of the blending ratio, a pressure regulator may be arranged between a gas supply and the respective first and/or second gas control valve for regulating the pressure of the supplied gas on the input side of the respective gas control valve.

A pressure sensor may be arranged upstream of the first and/or second gas control valve for measuring the pressure on the input side of the respective gas control valve, in particular to determine whether a gas is being supplied.

To increase the safety of using the gas blender an analyzer, preferably connected to a controller, may be present and arranged for analyzing the presence of a predetermined gas in at least one of the first and second gas control valves by correlating the opening status of the respective gas control valve with a gas flow on the output side of the respective gas control valve which is measured by a flow sensor, preferably by the internal flow sensor of a mass flow controller.

To be in a position to supply a medication to the blended gas flow a nebulizer may be in fluid communication with the pressure vessel, preferably via a pressure regulator and/or via a nebulizer gas control valve. A nebulizer gas control valve may be situated between the pressure vessel and the nebulizer to control the gas flow supplied to the nebulizer. The nebulizer gas control valve may be controlled by means of a controller on the basis of the gas demand of a patient, preferably on the basis of a respiratory pattern, preferably the respiratory frequency, determined by means of an analyzer for analyzing the respiratory pattern and associated parameters. In other words, the nebulizer may be controlled in an intermittent fashion such that medication is supplied only when the patient is inhaling.

In an alternative, the nebulizer gas control valve is a controllable valve, preferably a solenoid valve, and the flow to the nebulizer is adjusted on the basis of the pressure gradient determined on the basis of the pressure measured in the pressure vessel. The flow demanded by the nebulizer may be determined by means of the analysis of the pressure gradient which is determined on the basis of the pressure measured in the pressure vessel. In other words, by the determination of the pressure gradient present in the pressure vessel, the gas demand of the nebulizer can be determined such that different types or models of jet nebulizers can be connected to the gas blender and the gas blender sets the optimum parameters for operating the nebulizer automatically. In particular, on the basis of the analysis of the pressure gradient in the pressure vessel it can be determined whether a jet nebulizer is connected to the gas blender at all.

To further enhance the operability of the gas blender, it may include an analyzer for analyzing and/or displaying respiratory parameters of a patient, for example a flow curve of a patient, a respiratory peak flow value of a patient, a respiratory minute volume of a patient and/or a respiratory frequency of a patient on the basis of the opening status of the first and second gas control valves, on the basis of the pressure gradient measured in the pressure vessel and/or on the basis of a gas flow measured by at least one flow sensor arranged for sensing the flow, preferably the mass flow, of the first and/or the second gas into the pressure vessel, wherein the analyzer preferably is in communication with a controller. The pressure gradient in the pressure vessel is particularly suitable for determining the respiratory pattern and the associated parameters.

A controller may be arranged to simultaneously - or at least in an overlapping fashion - open and close the first and second gas control valves and preferably control the gas flow of the first and second gases to be constant when supplying the first and second gases into the pressure vessel. It is contemplated that the first and second gas control valves are operated at the same time but not consecutively in order to achieve a good degree of instantaneous mixing of the two gases in the pressure vessel.

The above mentioned objective is also solved by means of a method for blending at least two gases. The method comprises the steps of supplying a first gas via a first gas control valve into a pressure vessel, supplying a second gas via a second gas control valve into the pressure vessel, controlling the flows of the two gases by controlling the first and second gas control valves to obtain a predetermined blending ratio of the gases in the pressure vessel, and supplying the blended gas via an output gas control valve situated in fluid communication with the pressure vessel.

By providing the first gas and the second gas into the pressure cylinder, the pressure cylinder also provides for a buffer for the blended gas leading to high availability and a reasonably good constancy of supply of blended gas even in the cyclical applications when administering gases to a patient.

The blended gas may be supplied from the pressure cylinder via a demand valve, preferably a tilt demand valve. By using the control valve or the demand valve no gas is wasted which is to be supplied to a patient, but, at the same time, the pressure in the pressure vessel can be maintained at a suitable pressure range.

The pressure in the pressure vessel may be sensed and the first and second gas control valves opened for supplying gas into the pressure vessel if the pressure within the pressure vessel is below a predetermined lower threshold, and the first and second gas control valves closed if the pressure within the pressure vessel is above an upper threshold, the difference between the upper and the lower threshold preferably being in a range of between 0.01 and 2 bar, more preferred between 0.2 and 1.5 bar, most preferred between 0.5 and 1.4 bar.

The additional steps of sensing the flow, preferably the mass flow, of the first and/or the second gas into the pressure vessel by means of at least one flow sensor, and controlling, preferably via a controller, the first and/or second gas control valves on the basis of the gas flow sensed by the at least one flow sensor, may be present.

The accuracy in blending the gases may be further increased by the additional step of regulating the pressure of the gas supplied to the input side of the first and second gas control valves and/or determining the pressure on the input side of the first and second gas control valves.

The safety of using the method for blending the gases can be further enhanced by the additional steps of analyzing by means of an analyzer the presence of a predetermined gas in at least one of the first and second gas control valves by correlating the opening status of the respective gas control valve with a gas flow on the output side of the respective gas control valve sensed by a flow sensor.

The principle of this determination is relatively straightforward. For a known input pressure and a known output pressure the gas flow sensor expects a certain mass flow at a certain opening status of the gas control valve. For example, with the pressure differential between input and output pressure in mind, the flow rate for a gas is known when the control valve is fully opened or at any position between fully opened and closed. The mass flow under these conditions depends very much on the characteristics of the gas such that different mass flows would be measured for different gases at the same setting of pressures and opening status of the control valve.

Different gases differ, inter alia, in terms of their specific heat, viscosity, specific density and heat capacity ratio. These parameters can be used to determine, on the basis of the actual mass flow measured by the flow sensor and on the basis of the characteristics and opening status of the valve, whether a specific gas is flowing through the system.

Bearing in mind these settings, it becomes possible, when using a gas flow sensor and a gas control valve, to determine whether a specific gas, for example oxygen, is present and is flowing through the mass flow controller in the expected amount or whether a different gas is present.

This method and determination whether a specific gas present is highly relevant in medical applications. In particular, in situations in which oxygen is to be supplied to a patient, it is crucial that it is, indeed, oxygen that is supplied to the patient and, in particular, that it is avoided supplying hypoxic gases to the patient.

When administering a mixture of oxygen and helium to a patient, typically a cylinder containing a heliox blend, typically with 21% oxygen and 79% helium is supplied to the patient. In a second cylinder pure oxygen is present which can be blended with the heliox blend such that oxygen levels above 21% can be achieved and administered to the patient. This system is substantially failure safe because if one cylinder or one mixing valve fails, either pure oxygen or the heliox blend is supplied to the patient, which does not lead to life-threatening conditions because the patient is always supplied with a sufficient amount of oxygen.

In a different application of the gas blender, $CO_2$ and/or $N_2O$ can be blended with oxygen, with one another, with helium or with any other suitable gas or gas mixture.

However, it is contemplated exchanging the heliox supply by a supply containing pure helium, for example a cylinder of pure helium. In the gas blender described above it would then be possible to supply to the patient mixtures of helium and oxygen at a very flexible mixing rate. However, as it becomes immediately apparent, failure of the oxygen cylinder or of the oxygen supply would immediately lead to life-threatening complications if only helium were supplied to the patient. Accordingly, it is recommendable to determine whether any gas is flowing in the oxygen line and whether this gas is, indeed, oxygen. The method for determining the presence of a specific gas provides a reliable measure for this.

Furthermore, the method could obviate the need for a separate oxygen sensor. Oxygen sensors are difficult to maintain because they need to be adjusted from time to time and need to be replaced approximately every two years. Due to these complications using oxygen sensors in breathing systems is relatively cumbersome. However, with the method described above, an oxygen sensor would not be necessary anymore because it can be determined whether oxygen is present. The mass flow controller can also correctly control the amount of oxygen which will be present in the blended breathing gas. Accordingly, the method leads to a safe mode of operation of the above mentioned gas blender.

To be in a position to administer to a patient a medication, the additional step of providing a nebulizer with gas from the pressure vessel may be present, preferably via a pressure regulator and/or via a nebulizer gas control valve. The additional step of controlling the gas supply to the nebulizer on the basis of the gas demand of a patient, preferably on the basis of the respiratory frequency, by means of a gas control valve situated between the pressure vessel and the nebulizer may also be contemplated. In other words, the nebulizer may be controlled in an intermittent fashion such that medication is supplied only when the patient is inhaling.

To further enhance the treatment of a patient the additional step of analyzing and/or displaying by means of an analyzer respiratory parameters of a patient is contemplated, in particular a respiratory flow curve of a patient, a respiratory peak flow value of a patient, a respiratory minute volume of a patient and/or a respiratory frequency of a patient on the basis of the opening status of the first and second gas control valves, on the basis of the pressure gradient measured in the pressure vessel and/or on the basis of a gas flow measured by at least one flow sensor arranged for sensing the flow, preferably the mass flow, of the first and/or the second gas into the pressure vessel. The pressure gradient in the pressure vessel is particularly suitable for determining the respiratory pattern and the associated parameters.

These variations, modifications, alternatives, and alterations of the various preferred embodiments, arrangements and configurations may be used alone or in combination with one-another as will become more readily apparent to those with skill in the art, with reference to the following detailed description of the preferred embodiments and accompanying Figures and drawings.

Providing an analyzer, preferably connected to a controller, arranged for analyzing the presence of a predetermined gas in a gas control valve by correlating the opening status of the respective gas control valve with a gas flow on the output side of the respective gas control valve measured by a flow sensor is contemplated. Preferably, the gas control valve and the flow sensor are integrated in a mass flow controller.

A medical device for supplying a gas to a patient including a pressure vessel for receiving and storing a gas at above atmospheric pressure, an output gas control valve for supplying the gas from the pressure vessel to the patient and a nebulizer in fluid communication with the pressure vessel, preferably via a pressure regulator and/or via a nebulizer gas control valve, is further contemplated.

Furthermore, a medical device for supplying a gas to a patient via a gas control valve, including an analyzer for analyzing and/or displaying respiratory parameters of a patient, in particular a respiratory flow value of a patient, a respiratory peak volume of a patient, a respiratory minute volume of a patient and/or a respiratory frequency of a patient on the basis of the opening status of the gas control valve and/or on the basis of a gas flow measured by at least one flow sensor arranged for sensing the flow, preferably the mass flow, of the supplied gas, is also contemplated.

Brief description of the drawings

In the following, the present disclosure is exemplified by means of the drawings and Figures in which:

Figure 1    is a schematic diagram of a gas blender according to the present disclosure;

Figure 2    is a schematic diagram of a gas blender according to another embodiment of the present disclosure;

Figure 3    is yet another schematic diagram of a another gas blender according to the present disclosure;

Figure 4    is a detailed view of a demand valve;

Figure 5    is a schematic diagram of a gas blender according to the preset disclosure in another embodiment;

Figure 6    is a schematic diagram showing the discrimination of different gases; and

Figure 7    is a schematic diagram showing the operation of a gas blender according to the present disclosure.

Detailed description of the Figures

In the following description of exemplary embodiments of the present disclosure in the different Figures the same reference numerals are used for identical or similar features and repeated description thereof may be omitted in order to reduce redundancy.

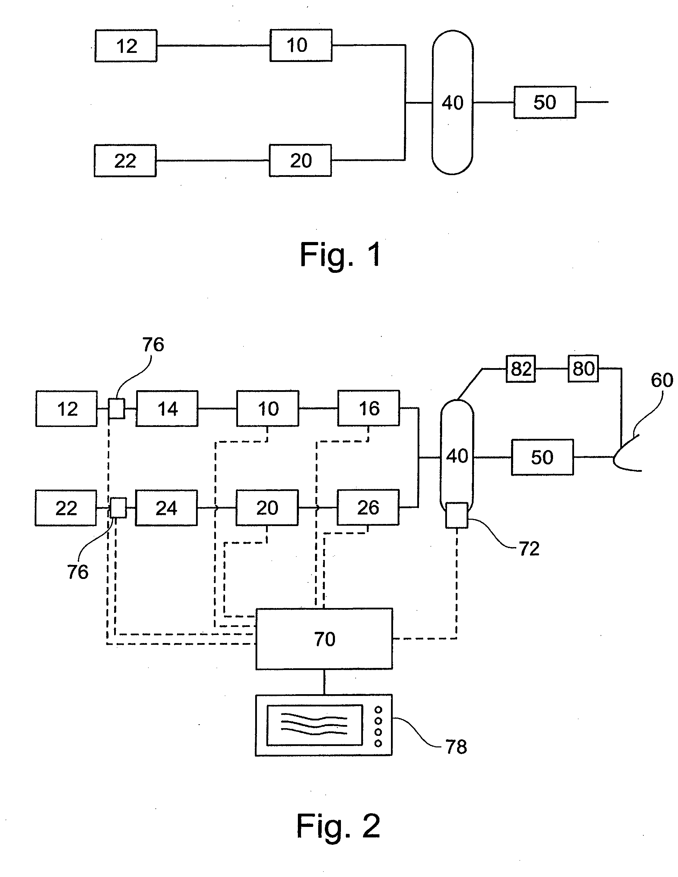

Figure 1 is a schematic diagram of a gas blender according to the present disclosure.

A first gas supply 12 is provided, for example a cylinder of oxygen ($O_2$) or a wall outlet of oxygen in a hospital. The gas provided by the gas supply 12 is delivered to a first gas control valve 10 from which it is supplied to a pressure vessel 40. The pressure vessel 40 is in fluid communication with the first gas control valve 10 and receives the gas supplied by the gas supply 12 via the gas control valve 10.

A second gas is supplied to the pressure vessel 40 in a manner similar to the first gas: A second gas supply 22 is present, for example a cylinder of a heliox blend, namely a mixture of 21% oxygen ($O_2$) and 79% helium (He). The pressure vessel 40 is in fluid communication with the second gas control valve 20 and receives the gas supplied by the second gas supply 22 via the gas control valve 20.

An output gas control valve 50 is in fluid communication with the pressure vessel 40 wherein the output gas control valve 50 is intended to supply the blended gas from the pressure vessel 40 to its intended use, for example for administering the gas to a patient.

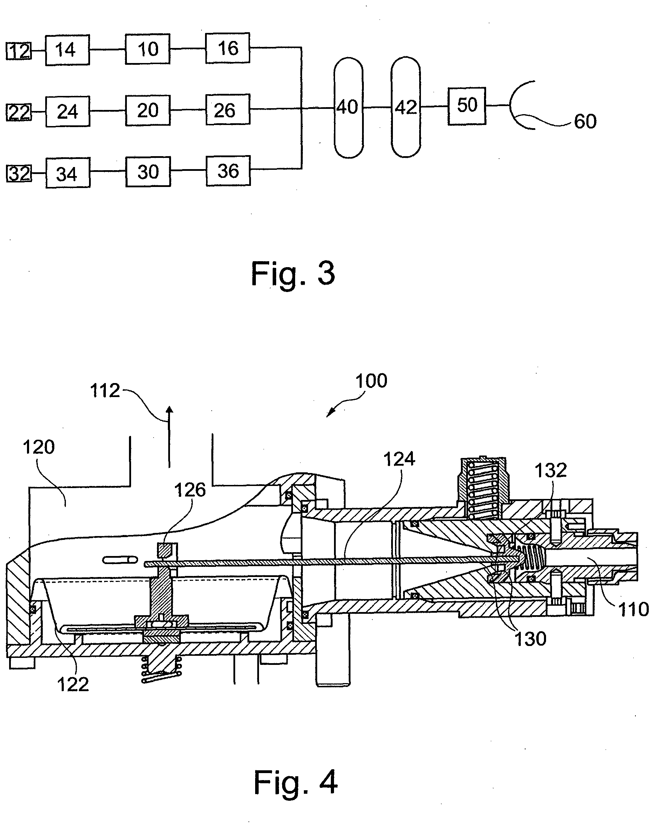

The output gas control valve 50 preferably is a demand valve which supplies the gas blend to a patient or any other user only on demand. Demand valves are known. The function of an exemplary tilt demand valve will be described with reference to Figure 4 below.

The first and the second gas control valves may be provided in the form of electric, hydraulic or pneumatic valves, preferably proportional valves, which can be controlled as to their opening status by means of electric, hydraulic or pneumatic signals. It is also contemplated using mass flow controllers as the first and second gas control valves. This latter option will be further elaborated below.

The pressure vessel 40 is intended to receive and store the first and second gases supplied to it by means of the first and the second gas control valves 10, 20. The gases are received and stored at above atmospheric pressure, in the given example at a working point of about 3 bar. Other suitable pressures are also contemplated wherein the pressures depend, inter alia, on the volume of the pressure vessel 40 as well as on the demand of the user, in particular on the demand of a patient, as well as on the capacity of the first and second gas control valves 10, 20 which "refill" the pressure vessel 40 when necessary.

The pressure vessel 40 may be provided in the form of a pressure cylinder having a suitable volume, for example 0.4 1. The volume of the pressure vessel 40 is chosen such that it provides a sufficient buffer for a blended gas to be administered to a patient. As the average tidal volume of an adult in rest is about 0.5 l, a pressure vessel with a volume of 0.4 1 provides at a pressure of 3 bar a usable volume of 0.8 1 as the gas is typically supplied to the patient at ambient pressure. In other words, in this example the pressure vessel 40 has a volume to buffer gas for at least one inhalation of a patient. The buffer volume in the pressure vessel 40 is chosen depending on several design considerations such as the accuracy of the blending ratio to be achieved in the pressure vessel over time, the tidal volume of a patient and the actual construction volume of the pressure vessel in a medical device.

By supplying the first and the second gases to the pressure vessel 40 via the first and second gas control valves 10, 20, the first gas and the second gas are blended in the pressure vessel 40. By supplying the first and second gases to the pressure vessel 40 in a predetermined ratio, a predetermined blending ratio of the gases can be achieved. In order to achieve the predetermined blending ratio of the gases, the first and the second gas control valves 10, 20 are controlled such that the respective mass flows of the first and second gases into the pressure vessel 40 corresponds to the predetermined blending ratio.

Controlling the mass flow of the first and second gases such that the predetermined blending ratio is reached can be achieved, in one example, by controlling the opening status of the respective gas control valves along a control curve of the valves wherein this control curve is determined beforehand for the respective gases and for the pressure environment present in the gas blender, i.e. the pressure of the input side of the respective gas control valve as well as the pressure on the output side of the gas control valve. The pressure of the gases on the input side of the gas control valves 10, 20 is known as it is regulated by the pressure regulators 14, 24. Of course, the presence of the pressure regulators 14, 24 is not mandatory, however, supplying the gas mass flow into the pressure vessel 40 can be carried out even more precisely if the pressure upstream of the first and second control valves is known and maintained. Because the first and second gas control valves 10, 20 are connected, on their output sides, to the pressure vessel 40, the pressure on the output side is controlled to vary only in a small range, for example between 2.5 bar and 3.0 bar or between 2.8 bar to 3.0 bar.

Accordingly, by the provision of the pressure vessel 40, the pressure conditions in the system are well determined such that controlling the first and second gas control valves simply along a control curve leads to improved accuracy of the supply of a predetermined mass flow of the gases to the pressure vessel 40.

In order to achieve an even higher accuracy, the first and second gas control valves 10, 20 are preferably opened and closed synchronously and the mass flow of the gases during supply thereof into the pressure vessel 40 is preferably kept constant over time. In other words, the first and second gas control valves 10, 20 are opened synchronously to an opening status (for example 50% opening) and are then kept at this opening status until they are synchronously closed again. The desired opening status may be determined on the basis of the control curve. By means of synchronously opening the first and second gas control valves 10, 20, or at least by opening the valves in an overlapping manner, the two different gases supplied to the pressure vessel 40 are reliably mixed.

The pressure vessel 40 is controlled to contain the gas blend in a limited pressure range only, for example in a range of between 2.5 bar and 3.0 bar, or between 2.8 bar and 3.0 bar. In order to maintain the pressure in the pressure vessel 40 the first and second gas control valves 10, 20 are controlled accordingly. In particular, soon as the intended pressure in the pressure vessel 40 is reached, or a predetermined upper threshold is reached, the first and second gas control valves 10, 20 are closed such that they do not supply any additional gas into the pressure vessel 40. If the pressure in the pressure vessel 40 drops below a predetermined lower threshold, the first and second gas control valves 10, 20 are opened again to supply gas at the predetermined blending ratio into the pressure vessel 40 until the intended pressure in pressure vessel 40 is reached again.

In the embodiment of Figure 2, which includes the same features as that of Figure 1 described above, additional features are shown as will be specified below.

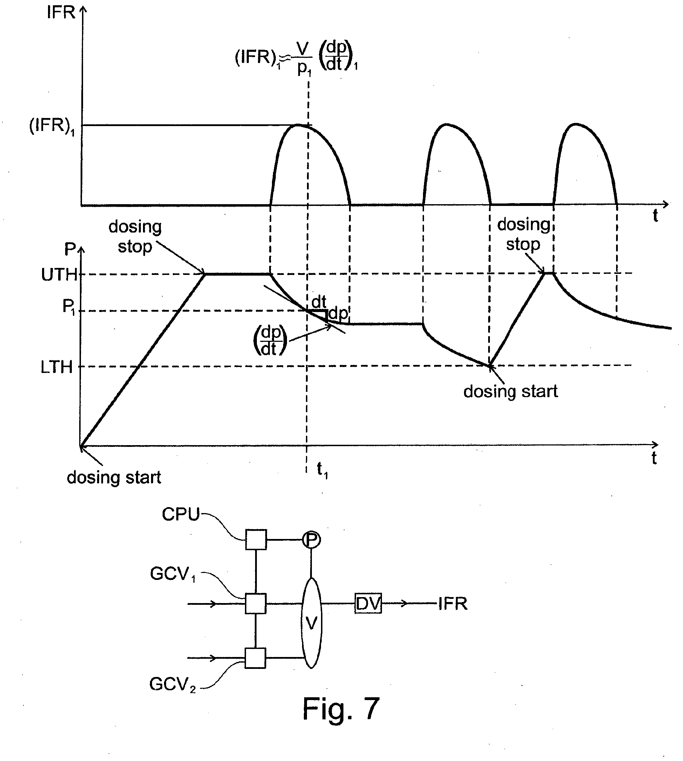

In order to control the pressure inside the pressure vessel 40, a pressure sensor 72 is connected thereto. The pressure sensor 72 may be either directly connected to the first and second gas control valves 10, 20 in the form of a simple ON/OFF-switch, or, as it is shown in Figure 2, it is connected to a controller 70 which controls the operation of the first and second gas control valves 10, 20. The controller 70 determines, via the pressure sensor 72, the pressure and opens the first and second gas control valves 10, 20 according to the predetermined mixing ratio as soon as the pressure drops below a lower pressure threshold and closes the first and second gas control valves 10, 20 as soon as an upper pressure threshold is exceeded. This principle will be further elaborated with respect to Figure 7 below.

The embodiment according to Figure 2 further includes, between the first and second gas supplies 12, 24, a first and second pressure regulator 14, 24 for regulating the pressure of the supplied gases from a supply pressure down to a predetermined value, for example down to 3 bar. The thus regulated first gas is supplied to a first gas control valve 10 which is in fluid communication with the first gas supply 12 via the pressure regulator 14. The second pressure regulator 24 regulates the pressure of the supplied second gas from the supply pressure down to a predetermined value, for example down to 3 bar. The second gas supply 22 is in fluid communication with a second gas control valve 20 via the pressure regulator 24.

The output gas control valve 50 is in fluid communication with the pressure vessel 40 wherein the output gas control valve 50 is intended to output the blended gas from the pressure vessel 40 to a mask 60 which is used for administering the gas blend to a patient. In place of the mask shown in Figure 2, any other patient interface, for example in the form of a nasal mask, a full face mask, a CPCP mask, an anaestetic face mask and/or a mouth piece is likewise contemplated to be used.

To supply the blended gas to a patient, the mask 60 is fitted to the mouth and nose of the patient and the patient starts breathing. The demand valve 50 automatically supplies the correct amount of gas through the mask 60 to the patient without wasting any gas. Because the pressure vessel 40 is at a predetermined pressure, the gas flow to the patient 60 can be kept at a constant pressure and constant flow rate.

Another set of pressure sensors 76 might be present at the fluid connection between the first and second gas supplies 12, 22 and the first and second pressure regulators 14, 24 in order to determine whether a gas is present at the desired pressure.

In order to provide a predetermined degree of humidity and/or to add medication to the gas that is going to be administered to a patient, a nebulizer 80 may be present which supplies the aerosol generated therein directly to the mask 60. Accordingly, the aerosol is added to the blended gas supplied by the output gas control valve 50 to the patient such that the patient receives the intended medication and/or degree of humidity of the gas.

The nebulizer 80 receives the high pressure gas from the pressure vessel 40 via a nebulizer gas control valve 82. Accordingly, the gas which is used to feed the nebulizer 80 corresponds to the gas blend intended to be supplied to the patient. Furthermore, the need for a separate high pressure supply of gas for feeding the nebulizer 80 is obviated.

The nebulizer gas control valve 82 is set such that the nebulizer receives the gas at the predetermined pressure which is necessary to reliably operate the nebulizer 80. The gas pressure results in a predetermined flow supplied to the nebulizer 80 which is dependent on the specific density of the supplied gas.

The nebulizer gas control valve 82 is a controllable valve, preferably a solenoid valve, and the flow to the nebulizer 80 is adjusted on the basis of the pressure gradient determined on the basis of the pressure measured in the pressure vessel 40. The flow demanded by the nebulizer may be determined by means of the analysis of the pressure gradient which is determined on the basis of the pressure measured in the pressure vessel. By the determination of the pressure gradient present in the pressure vessel 40, the gas demand of the nebulizer 80 can be determined such that different types or models of jet nebulizers 80 can be connected to the gas blender and the gas blender sets the optimum parameters for operating the nebulizer 80 automatically. In particular, on the basis of the analysis of the pressure gradient in the pressure vessel 40 it can be determined whether a jet nebulizer 80 is connected to the gas blender at all.

In a preferred embodiment, the nebulizer gas control valve is controlled such that gas is supplied to the nebulizer 80 only if the patient inhales and the gas supply to the nebulizer 80 is switched off if the patient exhales, in order to safe medication and gas in the nebulizer 80 and gas from the pressure vessel 40. This switching cycle of the nebulizer control valve can be triggered by determining via the pressure sensor 72 in the pressure vessel 40 the respiratory frequency and phase and controlling the nebulizer gas control valve accordingly.

In order to further improve the accuracy of the gas blend in the pressure cylinder 40, first and second flow sensors 16, 26 are provided on the output side of the first and second gas control valves 10, 20. The first and second flow sensors 16, 16 preferably are mass flow sensors which sense the mass flow of the first and second gases emitted on the output side of the respective first and second gas control valves 10, 20. Accordingly, the exact mass flow of the first and second gases which are supplied into the pressure vessel 40 can be measured and the first and second gas control valves 10, 20 can be controlled accordingly in order to maintain the desired blending ratio between the two gases.

In a preferred embodiment, the first gas control valve 10 and the first mass flow sensor 16 are integrated into one device, namely into a mass flow controller, i.e. the first gas control valve 10 and the first mass flow sensor 16 make up a first mass flow controller. The second gas control valve 20 and the second mass flow sensor 26 may also be integrated into a second mass flow controller.

In this disclosure, the term "mass flow controller" is understood to relate to a device used to measure and control the flow of fluids, in particular gases. A mass flow controller is designed and calibrated to control a specific type of fluid at a particular range of flow rates. The mass flow controller can be given a setpoint from 0 to 100% of its full scale range. The device will then control the rate of flow to the given setpoint.

All mass flow controllers have an inlet port, an outlet port, a mass flow sensor and a proportional control valve. A mass flow controller is fitted with a closed loop control system which is given an input signal by the operator (or an external circuit/computer) that it compares to the value from the mass flow sensor and adjusts the proportional valve accordingly to achieve the required flow.

Mass flow controllers require the supply gas to be within a specific pressure range. Low pressure will starve the mass flow controller of gas and it may fail to achieve its setpoint. High pressure may cause erratic flow rates.
As the mass flow sensor, a thermal mass flow meter may be used as it is customary for gas flow applications. As the name implies, thermal mass flow meters use heat to measure flow. Thermal mass flow meters introduce heat into the flow stream and measure how much heat dissipates using one or more temperature sensors. This method works best with gas mass flow measurement. It is difficult to get a strong signal using thermal mass flow meters in liquids, due to considerations relating to heat absorption.

While all thermal mass flow meters use heat to make their flow measurements, there are two different methods for measuring how much heat is dissipated. One method is called the constant temperature differential. Thermal mass flow meters using this method have two temperature sensors - a heated sensor and another sensor that measures the temperature of the gas. The mass flow rate is computed based on the amount of electrical power required to maintain a constant difference in temperature between the two temperature sensors. A second, and less popular concept, is called a constant current method.

Thermal mass flow meters using this method also have a heated sensor and another one that senses the temperature of the flow stream. The power to the heated sensor is kept constant. Mass flow is measured as a function of the difference between the temperature of the heated sensor and the temperature of the flow stream. Both methods are based on the principle that higher velocity flows result in a greater cooling effect. Both measure mass flow based on the measured effects of cooling in the flow stream.

Figure 3 shows, schematically, a third embodiment of the present blender for blending at least three different gases.

A first and a second gas supply 12, 22 as well as a third gas supply 32 are present. The line of the third gas supply 32 is, in principle, identical to the other two lines which are, in principle, identical to the gas blender shown in Figure 2.

In particular, a pressure regulator 34, a third gas control valve 30 and a flow sensor 36 are present. The third gas control valve 30 is in fluid connection with the pressure vessel 40. In the embodiment shown in Figure 3 a second pressure cylinder 42 is present in order to increase the working volume of the pressure cylinders.

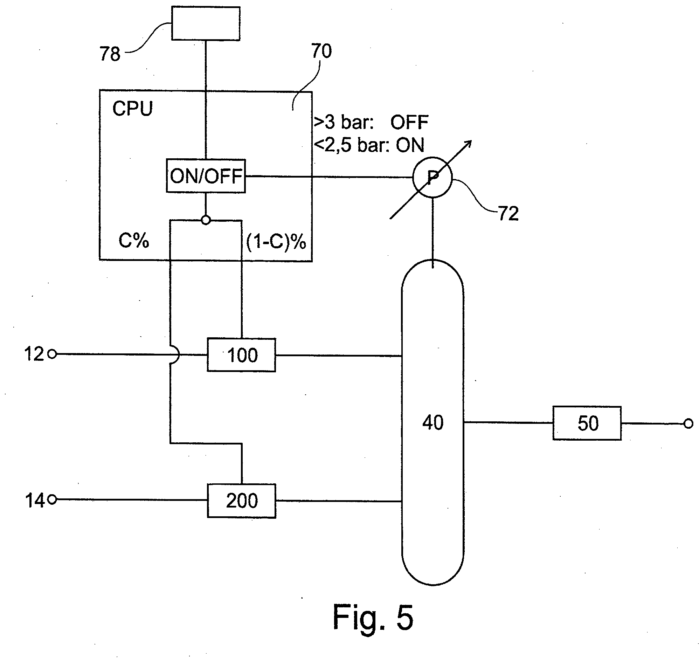

In Figure 5, another embodiment of a gas blender is shown which uses, according to the concept discussed above, a first and a second mass flow controller 100, 200 in place of the first and the second gas control valves and the first and second gas flow sensors. Accordingly, the first and second mass flow controllers 100, 200 comprise not only a proportional control vent but also a mass flow sensor and a closed control loop.

By means of the first and second mass flow controllers 100, 200, adjusting the correct gas mixture can be even more easily achieved in the pressure vessel 40 by setting the corresponding mass flows of the two different gases at the desired ratio. As mass flow controllers are substantially self-contained units which adjust the mass flow of a gas in a closed loop, the mixing ratio of the blended gas in the pressure vessel 40 can be maintained with high accuracy. The two gases with the controlled mass flow are supplied to the pressure vessel 40.

Mass flow controllers 100, 200 are very accurate as to the mass of the gases which they supply, provided they are operated at their respective optimum setpoint, i.e. in the optimum pressure regime. By means of the pressure vessel 40 it becomes possible to operate the mass flow controllers 100, 200 at their optimum setpoint. The reason for that is that the pressure in the pressure vessel 40 can be maintained in a narrow pressure band such that it is almost always filled up to a predetermined pressure (above atmospheric pressure) which corresponds to the optimum setpoint of the mass flow controllers 100, 200.

Preferably, the pressure in the pressure vessel 40 is controlled in a relatively narrow range, for example in a pressure range of +/- 0.2 bar above and below the optimum setpoint of the respective mass flow controllers 100, 200. Preferably, the mass flow controllers are selected such that their respective optimum setpoints are identical.

In order to improve the handling of the devices discussed above, the controller 70 may be connected to a digital display 78 which enables the user to set at least the desired mixing ratio on the display.

It is contemplated, in medical applications, to replace the heliox mixture used at gas supply 22 by a pure helium cylinder in order to increase the packing density and reduce costs for the user (i.e. the hospital). Accordingly, the required heliox mixture is then mixed from pure helium and pure oxygen. However, as will be appreciated, it could be fatal for the patient if the oxygen supply failed or a wrong cylinder would be connected to the oxygen connector such that a hypoxic gas would be administered to the patient. It is crucial for the operation of a medical device that it is ensured that a hypoxic gas is never administered to a patient. Accordingly, it is important to know whether the intended gas actually flows through the first and second gas control valves 10, 20.

Accordingly, it would be an option to use an oxygen sensor either in the supply lines and/or in the tube leading to the mask of the patient in order to determine whether the oxygen content of the gas administered to the patient is sufficient. However, using electrochemical oxygen sensors is relatively cumbersome because they have the tendency to drift and, thus, need to be adjusted frequently in order to work reliably. Furthermore, oxygen sensors need to be replaced every now and then in order to ensure their proper operation.

However, in order to determine whether the intended gas is supplied at the first and second gas supplies 12, 22, it is possible to run at least a plausibility check by means of the first and second gas control valves 10, 20 in combination with the respective first and second gas flow sensors 16, 26. In an alternative, the mass flow controllers 100, 200 can likewise be used for the plausibility check. Accordingly, in addition to the operation described above, it is possible to use the first and second gas control valves 10, 20 in combination with the first and second gas flow sensors 16, 26 not only for controlling the exact blending ratio of the gases, but also to determine whether the correct gases are supplied by the supplies 12, 22.

To this end, the presence of a specific gas can be determined by correlating the opening status of the gas control valve with the gas flow measured in the gas flow sensor. For a specific gas, for example oxygen, the flow sensor signal output by the gas flow sensor corresponds to a certain opening status of the gas control valve for a known pressure differential between its upstream end and downstream end. In other words, if oxygen is supplied to the first gas control valve 10 at a certain pressure regulated by the pressure regulator 14, a certain flow sensor signal measured by the first gas flow sensor 16 is expected for a certain opening status of the first gas control valve 10. If a different gas would flow through the first gas control valve 10 as well as through the gas flow sensor 16, a flow sensor signal different from the flow sensor signal expected for the oxygen gas flow would be measured by the gas flow sensor 16 at the identical opening position of the first gas control valve 10.

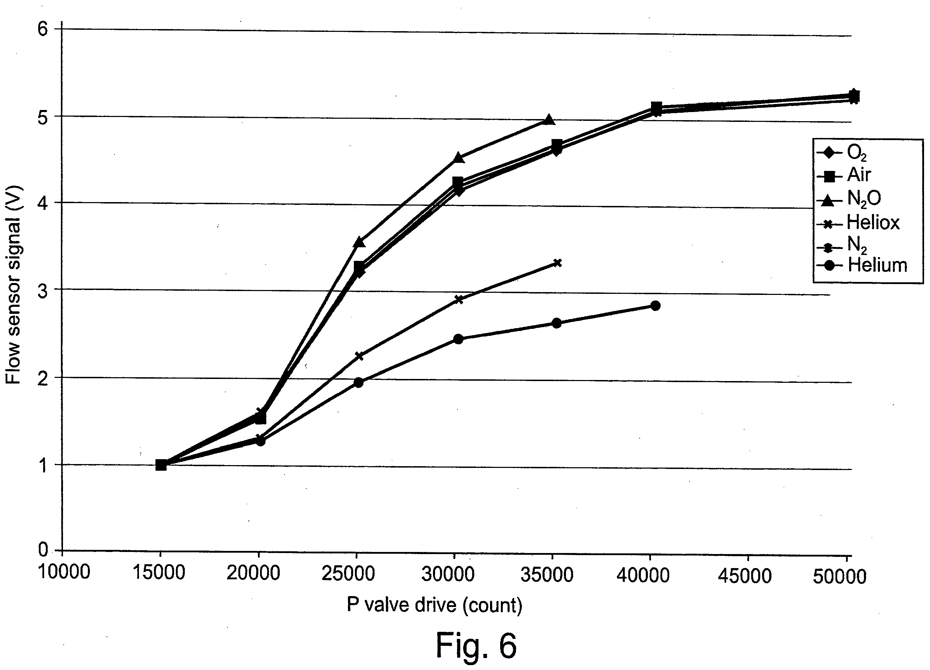

This is shown, schematically, in Figure 6 in which the flow sensor signal of different gases measured with Honeywell AWM720P1 flow sensor is shown in relation to the opening status (corresponding to the supply voltage) of a proportional electric vent (ASCO U6202NV24DC). As can be immediately seen, the flow sensor signal is different at the same opening status of the valve for different gases. Thus, it becomes possible to determine whether a specific (predetermined) gas is present at a gas control valve if the gas flow measured on its output side is correlated or compared with the expected gas flow.

In other words, the first and second gas control valves in combination with the first and second gas flow sensors, or a first and second mass flow controller, can be used for determining whether a predetermined gas is present or absent.

It is even possible determining which other gas is used by correlating the mass flow measured with the opening status of the control valve and comparing this with known values. However, the main concern is to determine whether or not the intended gases are flowing through the first and second gas control valves.

According to the present disclosure this determination can be achieved by using the method outlined above, namely by measuring the gas flow through the gas control valve and correlating this measurement with the opening status of the gas control valve. This determination may further be improved by measurements provided by additional pressure sensors situated before and after the gas control valves 10, 20.

The pressure in the pressure vessel 40 sensed by the pressure sensor 72 over time in combination with the knowledge of the volume supplied to the pressure vessel 40 via the first and second gas control valves 10, 20 can also be used to analyze, in an analyzer, different respiratory parameters of the patient connected to the gas blender.

As a rough estimation, the flow can be determined on the basis of the following equation:

$$Flow \ = \ (V/p)*(dp/dt)$$

Wherein V is the volume of the pressure vessel 40, p is the temporary pressure in the pressure vessel, and dp/dt is the pressure gradient.

Accordingly, on the basis of the pressure in the pressure vessel 40, compensated for the added volume from the first and second gas control valves 10, 20, it is possible to analyze and/or display a flow curve, i.e. the flow over time. The peak flow value of the inhalation can be determined and can be displayed to visualize a trend of the peak flow values. The respiratory minute volume can be determined, e.g. by integrating the flow curve or by determining it on the basis of the gas volumes supplied into the pressure vessel via the first and second gas control valves 10, 20. The onset of the inhalation can be determined and the respiratory frequency can be determined and displayed. The onset of the inhalation can be determined and be used to open up a nebulizer gas control valve 82 such as to feed a nebulizer only when the patient inhales and at the end of the inhalation, the nebulizer gas control valve 82 is switched off again.

Figure 4 shows, schematically, a demand valve which is to be connected to the output gas control valve 50 of the pressure vessel 40 at connector 110. At reference numeral 112, the gas blend is supplied to the patient.

As soon as the patient starts inhaling, a slight under-pressure is generated in chamber 120 such that the membrane 122 moves towards the under-pressure, i.e. upwards in Figure 3. A lever 124 is connected to the membrane via a follower 126 such that when the membrane 122 moves upwards, the lever 124 is operated accordingly. As soon as the lever 124 is moved, the valve 130 is opened, pivoting around the pivoting point 132.

The demand valve opens the fluid connection between the pressure vessel 40, which is connected to connector 110, and the patient connected at reference numeral 112 as long as the patient inhales. If the patient stops inhaling and, thus, a slight over-pressure accrues in chamber 120, the membrane 122 moves back into the position shown in Figure 4 such that the valve 130 is closed again.

Figure 7 shows, in an example, the control principle for controlling the supply of the first and second gases into the pressure vessel. At the bottom of the Figure, the principle of the circuit on the basis of which the function of the control will be explained, is shown. In particular, a first gas control valve GCV1 and a second gas control valve GCV2 are provided which supply gas into the pressure vessel indicated by the volume V. The gas is then supplied to the user via a demand valve DV. The gas control valves GCV1 and GCV2 are controlled by a controller CPU which also receives pressure measurements from a pressure sensor P which is connected to the volume of the pressure vessel V.

In the upper portion of the Figure, a first diagram is shown which depicts, schematically, the inspiratory flow curve of a patient, in particular the inspiratory flow rate (IFR), over time t.

In the middle portion of the Figure, a second diagram is shown which depicts, schematically, the pressure p inside of the pressure vessel over time t. In other words, the upper diagram shows the gas demand and the lower diagram shows the pressure in the pressure vessel. The first and the second diagram are linked with respect to their time bases.

At the time point "dosing start", the controller CPU opens the two gas control valves GCV1 and GCV2 according to the predetermined blending ratio. Accordingly, the pressure in the pressure vessel rises until the upper threshold (UTH) is reached. When the upper threshold UTH is reached, the two gas control valves are closed, as is indicated at "dosing stop".

When the patient starts inhaling, as is shown by the first inhaling cycle, gas from the pressure vessel is withdrawn and the pressure in the pressure vessel drops. However, as the lower threshold (LTH) has not yet been reached, the controller CPU keeps the first and second gas control valves GCV1, GCV2 shut.

At the end of the second inhaling cycle of the patient, however, the lower threshold LTH is reached such that the controller CPU opens the first and second gas control valves GCV1, GCV2 at the time point "dosing start". New gas is supplied into the pressure vessel until the upper threshold UTH is reached and the controller CPU shuts the first and second gas control valves GCV1, GCV2 at the time point "dosing stop" again. By means of this principle, constant supply of the blended gas to the patient can be achieved.

By determining the slope of the pressure curve in the pressure vessel it becomes possible to determine the respiratory pattern of the patient. In the diagrams of Figure 7 this is shown, in an exemplary fashion, at time point $t_1$. At this time point, the slope dp/dt of the pressure curve p(t) is determined. On the basis of the disclosure above, the inspiratory flow rate at this time point can then be calculated on the basis of the equation

$$(IFR)_1 \ = \ (V/p_1)*(dp/dt)_1,$$

such that the respiratory pattern can be determined over time by simply measuring the pressure p in the pressure vessel V. Of course, corrections need to be made for any dosing of gas into the pressure vessel which occurs during an inhalation cycle or for any gas that is supplied to a nebulizer.

In the following, the present disclosure is summarized with respect to some of its embodiments:

1. Gas blender for blending at least two different gases, the gas blender comprising

- a first gas control valve (10) for supplying a first gas,
- a second gas control valve (20) for supplying a second gas,
- a pressure vessel (40) in fluid communication with the first and second gas control valves (10, 20) to receive and store the first and second gases at above atmospheric pressure, and
- an output gas control valve (50) in fluid communication with an output of the pressure vessel for supplying the blended gas from the pressure vessel.

2. Gas blender according to 1, wherein the output gas control valve is a demand valve (50), preferably a tilt demand valve, sensing the gas demand of a patient and supplying the blended gas accordingly.

3. Gas blender according to 1 or 2, wherein the volume of the pressure vessel (40) is in the range of 0.01 l to 10 l, preferably 0.1 l to 3 l, more preferred 0.2 l to 0.5 l, most preferred 0.4 l.

4. Gas blender according to any one of the preceding embodiments, wherein the maximum flow rate of the first and/or second gas control valve is set to between 5 l/min and 350 l/min, preferably to between 10 l/min to 200 l/min, more preferred to between 140 l/min to 160 l/min, most preferred to 150 l/min.

5. Gas blender according to any one of the preceding embodiments, comprising a controller (70) connected to the first and second gas control valves (10, 20) for controlling the operation of the first and second gas control valves (10, 20) on the basis of a predetermined blending ratio.

6. Gas blender according to any one of the preceding embodiments, comprising a pressure sensor (72) for sensing the pressure in the pressure vessel (40), the pressure sensor being connected, preferably via a controller (70), to the first and second gas control valves for controlling the supply of the first and second gas into the pressure vessel.

7. Gas blender according to any one of the preceding embodiments, including at least one flow sensor (16, 26) arranged for sensing the flow, preferably the mass flow, of the first and/or the second gas into the pressure vessel, the flow sensor being connected, preferably via a controller (70), to the first and second gas control valves to control the first and/or second gas control valves on the basis of the gas flow measured by the at least one flow sensor.

8. Gas blender according to any one of the preceding embodiments, wherein the first and/or the second gas control valve is an electric, hydraulic or pneumatic gas control valve controllable by a controller (70) and/or wherein the first and/or second gas control valve is a mass flow controller (100, 200).

9. Gas blender according to any one of the preceding embodiments, wherein a gas pressure sensor (74) is arranged downstream of the output gas control valve (50) for sensing the gas pressure of the blended gas administered to a patient, the gas pressure sensor preferably being arranged between the output gas control valve (50) and a patient interface, preferably in the form of a mask (60), a nasal mask, a full face mask, a CPCP mask, an anaestetic face mask and/or a mouth piece for administering the gas to a patient.

10. Gas blender according to any one of the preceding embodiments, wherein a patient interface, preferably in the form of a mask (60), a nasal mask, a full face mask, a CPCP mask, an anaestetic face mask and/or a mouth piece for administering the blended gas to a patient is in fluid communication with the output side of the output gas control valve (50).

11. Gas blender according to any one of the preceding embodiments, wherein a pressure regulator (14, 24) is arranged between a gas supply (12, 22) and the respective first and/or second gas control valve (10, 20) for regulating the pressure of the supplied gas on the input side of the respective gas control valve (10, 20) and/or wherein a pressure sensor (76) is arranged upstream of the first and/or second gas control valve (10, 20) for measuring the pressure on the input side of the respective gas control valve (10, 20).

12. Gas blender according to any one of the preceding embodiments, wherein an analyzer, preferably connected to a controller (70), is arranged for analyzing the presence of a predetermined gas in at least one of the first and second gas control valves (10, 20) by correlating the opening status of the respective gas control valve with a gas flow on the output side of the respective gas control valve measured by a flow sensor (16, 26).

13. Gas blender according to any one of the preceding embodiments, wherein a nebulizer (80) is in fluid communication with the pressure vessel (40), preferably via a pressure regulator and/or via a nebulizer gas control valve.

14. Gas blender according to 13, wherein a nebulizer gas control valve is situated between the pressure vessel (40) and the nebulizer (80) and wherein the nebulizer gas control valve is controlled by means of a controller (70) on the basis of the gas demand of a patient.

15. Gas blender according to claim 13 or 14, wherein the nebulizer gas control valve (82) is a controllable valve, preferably a solenoid valve, and the flow to the nebulizer (80) through the nebulizer control valve (82) is adjustable on the basis of the pressure gradient determined on the basis of the pressure measured in the pressure vessel (40).

16. Gas blender according to any one of the preceding embodiments, wherein an analyzer is present for analyzing and/or displaying respiratory parameters of a patient, in particular a flow curve of a patient, a respiratory peak flow value of a patient, a respiratory minute volume of a patient and/or a respiratory frequency of a patient on the basis of the opening status of the first and second gas control valves (10, 20) and/or on the basis of a gas flow measured by at least one flow sensor (16, 26) arranged for sensing the flow, preferably the mass flow, of the first and/or the second gas into the pressure vessel, wherein the analyzer preferably is in communication with a controller (70).

17. Gas blender according to any one of the preceding embodiments, wherein a controller (70) is arranged to simultaneously open and close the first and second gas control valves (10, 20) and preferably control the gas flow of the first and second gases to be constant when supplying the first and second gases into the pressure vessel.

18. Method for blending at least two gases, comprising the steps:

- supplying a first gas via a first gas control valve (10) into a pressure vessel (40),
- supplying a second gas via a second gas control valve (20) into the pressure vessel (40),
- controlling the flows of the two gases by controlling the first and second gas control valves to obtain a predetermined blending ratio of the gases in the pressure vessel (40),
- supplying the blended gas via an output gas control valve (50) situated in fluid communication with the pressure vessel (40).

19. Method according to 18, comprising the step of supplying the blended gas from the pressure vessel (40) to a patient depending on the gas demand of the patient, preferably via an output gas control valve in the form of a demand valve, more preferred via a tilt demand valve.

20. Method according to 18 or 19, comprising the steps of sensing the pressure in the pressure vessel (40), opening the first and second gas control valves (10, 20) for supplying gas into the pressure vessel (40) if the pressure within the pressure vessel is below a predetermined lower threshold, and closing the first and second gas control valves (10, 20) if the pressure within the pressure vessel is above an upper threshold, the difference between the upper and the lower threshold preferably being in a range of between 0.01 and 2 bar, more preferred between 0.2 and 1.5 bar, most preferred between 0.5 and 1.4 bar.

21. Method according to any one of 18 to 20, comprising the additional steps of sensing the flow, preferably the mass flow, of the first and/or the second gas into the pressure vessel by means of at least one flow sensor, and controlling, preferably via a controller (70), the first and/or second gas control valves on the basis of the gas flow sensed by the at least one flow sensor.

22. Method according to any one of 18 to 21, comprising the additional steps of sensing the gas pressure of the blended gas administered to a patient by means of a gas pressure sensor (74) arranged downstream of the output gas control valve (50) and controlling, preferably via a controller (70), the first and second gas control valves (10, 20) on the basis of the gas pressure sensed.

23. Method according to any one of 18 to 22, comprising the step of regulating the pressure of the gas supplied to the input side of the first and second gas control valves (10, 20) and/or determining the pressure on the input side of the first and second gas control valves (10, 20).

24. Method according to any one of 18 to 23, comprising the additional steps of analyzing by means of an analyzer the presence of a predetermined gas in at least one of the first and second gas control valves (10, 20) by correlating the opening status of the respective gas control valve (10, 20) with a gas flow on the output side of the respective gas control valve sensed by a flow sensor (16, 26).

25. Method according to any one of 18 to 24, comprising the additional step of providing a nebulizer (80) with gas from the pressure vessel (40), preferably via a pressure regulator and/or via a nebulizer gas control valve.

26. Method according to 25, comprising the additional step of controlling the gas supply to the nebulizer on the basis of the gas demand of a patient by means of a gas control valve situated between the pressure vessel (40) and the nebulizer (80).

27. Method according to claim 25 or 26, wherein the nebulizer gas control valve (82) is a controllable valve, preferably a solenoid valve, and the flow to the nebulizer (80) through the nebulizer control valve (82) is adjusted on the basis of the pressure gradient determined on the basis of the pressure measured in the pressure vessel (40).

28. Method according to any one of 18 to 27, comprising the additional step of analyzing and/or displaying by means of an analyzer respiratory parameters of a patient, in particular a respiratory flow curve of a patient, a respiratory peak flow value of a patient, a respiratory minute volume of a patient and/or a respiratory frequency of a patient on the basis of the opening status of the first and second gas control valves (10, 20) and/or on the basis of a gas flow measured by at least one flow sensor (16, 26) arranged for sensing the flow, preferably the mass flow, of the first and/or the second gas into the pressure vessel.

29. Method according to any one of 18 to 28, wherein the first and the second gas control valve (10, 20) are opened and/or closed simultaneously and the gas flow over time of the first and the second gases preferably is constant.

30. Medical device using the gas blender and/or the method according to any one of the preceding embodiments.

31. Analyzer, preferably connected to a controller (70), arranged for analyzing the presence of a predetermined gas in a gas control valve (10, 20) by correlating the opening status of the respective gas control valve with a gas flow on the output side of the respective gas control valve measured by a flow sensor (16, 26).

32. Analyzer according to 31, wherein the gas control valve and the flow sensor are integrated in a mass flow controller (100, 200).

33. Method for analyzing the presence of a predetermined gas in a gas control valve (10, 20) by correlating the opening status of the gas control valve with a gas flow on the output side of the gas control valve sensed by a flow sensor (16, 26).

34. Medical device for supplying a gas to a patient including a pressure vessel (40) for receiving and storing a gas at above atmospheric pressure, an output gas control valve (50) for supplying the gas from the pressure vessel to the patient and a nebulizer (80) in fluid communication with the pressure vessel (40), preferably via a pressure regulator and/or via a nebulizer gas control valve.

35. Method of operating a medical device including the steps of supplying the gas to a patient from a pressure vessel via an output gas control valve (50) and supplying gas from the pressure vessel (40) to a nebulizer (80), preferably via a pressure regulator and/or via a nebulizer gas control valve.

36. Medical device for supplying a gas to a patient via a gas control valve (10, 20), including an analyzer for analyzing and/or displaying respiratory parameters of a patient, in particular a respiratory flow value of a patient, a respiratory peak volume of a patient, a respiratory minute volume of a patient and/or a respiratory frequency of a patient on the basis of the opening status of the gas control valve and/or on the basis of a gas flow measured by at least one flow sensor (16, 26) arranged for sensing the flow, preferably the mass flow, of the supplied gas.

37. Method for operating a medical device, comprising the steps of analyzing and/or displaying by means of an analyzer respiratory parameters of a patient, in particular displaying respiratory parameters of a patient, in particular a respiratory flow value of a patient, a respiratory peak volume of a patient, a respiratory minute volume of a patient and/or a respiratory frequency of a patient on the basis of an opening status of a gas control valve (10, 20) for supplying a gas to the patient and/or on the basis of a gas flow measured by at least one flow sensor (16, 26) arranged for sensing the flow, preferably the mass flow, of the gas supplied to the patient.

The scope of the invention is defined by the appended claims.

**Claims**

1. Gas blender for blending at least two different gases, the gas blender comprising

   - a first gas control valve (10) for supplying a first gas,
   - a second gas control valve (20) for supplying a second gas,
   - a pressure vessel (40) in fluid communication with the first and second gas control valves (10, 20) to receive and store the first and second gases at above atmospheric pressure, and
   - an output gas control valve (50) in fluid communication with an output of the pressure vessel for supplying the blended gas from the pressure vessel.

**2.** Gas blender according to claim 1, wherein the output gas control valve is a demand valve (50), preferably a tilt demand valve, sensing the gas demand of a patient and supplying the blended gas accordingly.

**3.** Gas blender according to claim 1 or 2, wherein the volume of the pressure vessel (40) is in the range of 0.01 l to 10 l, preferably 0.1 l to 3 l, more preferred 0.2 l to 0.5 l, most preferred 0.4 l.

**4.** Gas blender according to any one of the preceding claims, wherein the maximum flow rate of the first and/or second gas control valve is set to between 5 l/min and 350 l/min, preferably to between 10 l/min to 200 l/min, more preferred to between 140 l/min to 160 l/min, most preferred to 150 l/min.

**5.** Gas blender according to any one of the preceding claims, comprising a controller (70) connected to the first and second gas control valves (10, 20) for controlling the operation of the first and second gas control valves (10, 20) on the basis of a predetermined blending ratio.

**6.** Gas blender according to any one of the preceding claims, comprising a pressure sensor (72) for sensing the pressure in the pressure vessel (40), the pressure sensor being connected, preferably via a controller (70), to the first and second gas control valves for controlling the supply of the first and second gas into the pressure vessel.

**7.** Gas blender according to any one of the preceding claims, including at least one flow sensor (16, 26) arranged for sensing the flow, preferably the mass flow, of the first and/or the second gas into the pressure vessel, the flow sensor being connected, preferably via a controller (70), to the first and second gas control valves to control the first and/or second gas control valves on the basis of the gas flow measured by the at least one flow sensor.

**8.** Gas blender according to any one of the preceding claims, wherein the first and/or the second gas control valve is an electric, hydraulic or pneumatic gas control valve controllable by a controller (70) and/or wherein the first and/or second gas control valve is a mass flow controller (100, 200).

**9.** Gas blender according to any one of the preceding claims, wherein a nebulizer (80) is in fluid communication with the pressure vessel (40), preferably via a pressure regulator and/or via a nebulizer gas control valve (82).

**10.** Method for blending at least two gases, comprising the steps:

- supplying a first gas via a first gas control valve (10) into a pressure vessel (40),
- supplying a second gas via a second gas control valve (20) into the pressure vessel (40),
- controlling the flows of the two gases by controlling the first and second gas control valves to obtain a predetermined blending ratio of the gases in the pressure vessel (40),
- supplying the blended gas via an output gas control valve (50) situated in fluid communication with the pressure vessel (40).

**11.** Method according to claim 10, comprising the steps of sensing the pressure in the pressure vessel (40), opening the first and second gas control valves (10, 20) for supplying gas into the pressure vessel (40) if the pressure within the pressure vessel is below a predetermined lower threshold, and closing the first and second gas control valves (10, 20) if the pressure within the pressure vessel is above an upper threshold, the difference between the upper and the lower threshold preferably being in a range of between 0.01 and 2 bar, more preferred between 0.2 and 1.5 bar, most preferred between 0.5 and 1.4 bar.

**12.** Method according to claim 10 or 11, comprising the additional steps of sensing the flow, preferably the mass flow, of the first and/or the second gas into the pressure vessel by means of at least one flow sensor, and controlling, preferably via a controller (70), the first and/or second gas control valves on the basis of the gas flow sensed by the at least one flow sensor.

**13.** Method according to any one of claims 10 to 12, comprising the additional step of providing a nebulizer (80) with gas from the pressure vessel (40), preferably via a pressure regulator and/or via a nebulizer gas control valve.

**14.** Medical device for supplying a gas to a patient via a gas blender according to any one of claims 1 to 9, further including an analyzer for analyzing and/or displaying respiratory parameters of a patient, in particular a respiratory flow value of a patient, a respiratory peak volume of a patient, a respiratory minute volume of a patient and/or a respiratory frequency of a patient on the basis of the opening status of the gas control valve and/or on the basis of a gas flow measured by at least one flow sensor (16, 26) arranged for sensing the flow, preferably the mass flow, of the supplied gas.

**Patentansprüche**

**1.** Gasmischer zum Mischen von mindestens zwei unterschiedlichen Gasen, wobei der Gasmischer umfasst

- ein erstes Gasregelventil (10) zum Zuführen eines ersten Gases,
- ein zweites Gasregelventil (20) zum Zuführen eines zweiten Gases,
- einen Druckbehälter (40) in Fluidkommunikation mit dem ersten und dem zweiten Gasregelventil (10, 20) zum Aufnehmen und Lagern des ersten und des zweiten Gases oberhalb des Atmosphärendrucks, und
- ein Ausgangs-Gasregelventil (50) in Fluidkommunikation mit einem Ausgang des Druckbehälters zum Zuführen des gemischten Gases aus dem Druckbehälter.

2. Gasmischer nach Anspruch 1, wobei das Ausgangs-Gasregelventil ein Bedarfsventil (50), vorzugsweise ein kippbares Bedarfsventil ist, der den Gasbedarf eines Patienten misst und das gemischte Gas entsprechend zuführt.

3. Gasmischer nach Anspruch 1 oder 2, wobei das Volumen des Druckbehälters (40) im Bereich von 0,01 l bis 10 l, vorzugsweise von 0,1 l bis 3 l, besonders bevorzugt von 0,2 l bis 0,5 l, ganz besonders bevorzugt bei 0,4 l liegt.

4. Gasmischer nach einem der vorstehenden Ansprüche, wobei die maximale Flussrate des ersten und/oder zweiten Gasregelventils zwischen 5 l/min und 350 l/min, vorzugsweise zwischen 10 l/min und 200 l/min, besonders bevorzugt zwischen 140 l/min und 160 l/min, ganz besonders bevorzugt auf 150 l/min eingestellt wird.

5. Gasmischer nach einem der vorstehenden Ansprüche, der einen Regler (70) umfasst, der an das erste und das zweite Gasregelventil (10, 20) angeschlossen ist, um den Betrieb des ersten und des zweiten Gasregelventils (10, 20) anhand eines vorbestimmten Mischverhältnisses zu steuern.

6. Gasmischer nach einem der vorstehenden Ansprüche, der einen Drucksensor (72) zum Messen des Drucks im Druckbehälter (40) umfasst, wobei der Drucksensor vorzugsweise über einen Regler (70) an das erste und das zweite Gasregelventil angeschlossen ist, um die Zufuhr des ersten und des zweiten Gases in den Druckbehälter zu steuern.

7. Gasmischer nach einem der vorstehenden Ansprüche, der mindestens einen Durchflusssensor (16, 26) umfasst, der dazu eingerichtet ist den Fluss, vorzugsweise den Massenfluss, des ersten und/oder zweiten Gases in den Druckbehälter zu messen, wobei der Durchflusssensor vorzugsweise über einen Regler (70) an das erste und das zweite Gasregelventil angeschlossen ist, um das erste und/oder zweite Gasregelventil anhand des vom mindestens einen Durchflusssensor gemessenen Gasflusses zu steuern.

8. Gasmischer nach einem der vorstehenden Ansprüche, wobei das erste und/oder zweite Gasregelventil ein elektrisches, hydraulisches oder pneumatisches Gasregelventil ist, das über einen Regler (70) steuerbar ist, und/oder wobei das erste und/oder zweite Gasregelventil ein Massendurchflussregler (100, 200) ist.

9. Gasmischer nach einem der vorstehenden Ansprüche, wobei ein Vernebler (80) vorzugsweise über einen Druckregler und/oder ein Vernebler-Gasregelventil (82) in Fluidkommunikation mit dem Druckbehälter (40) steht.

10. Verfahren zum Mischen von mindestens zwei Gasen, das folgende Schritte umfasst:

- Zuführen eines ersten Gases über ein erstes Gasregelventil (10) in einen Druckbehälter (40),
- Zuführen eines zweiten Gases über ein zweites Gasregelventil (20) in den Druckbehälter (40),
- Steuern des Flusses der beiden Gase durch Steuern des ersten und des zweiten Gasregelventils, um ein vorbestimmtes Mischverhältnis der Gase im Druckbehälter (40) zu erzielen,
- Zuführen des gemischten Gases über ein in Fluidkommunikation mit dem Druckbehälter (40) stehendes Ausgangs-Gasregelventil (50).

11. Verfahren nach Anspruch 10, umfassend die Schritte Messen des Drucks im Druckbehälter (40), Öffnen des ersten und des zweiten Gasregelventils (10, 20), um Gas in den Druckbehälter (40) zu führen, wenn der Druck innerhalb des Druckbehälters unter einem vorbestimmten unteren Schwellenwert liegt, und Schließen des ersten und des zweiten Gasregelventils (10, 20), wenn der Druck innerhalb des Druckbehälters über einem oberen Schwellenwert liegt, wobei der Unterschied zwischen dem oberen und dem unteren Schwellenwert vorzugsweise im Bereich von 0,01 bis 2 bar, besonders bevorzugt von 0,2 bis 1,5 bar, ganz besonders bevorzugt von 0,5 bis 1,4 bar liegt.

12. Verfahren nach Anspruch 10 oder 11, umfassend die zusätzlichen Schritte Messen des Flusses, vorzugsweise des Massenflusses, des ersten und/oder zweiten Gases in den Druckbehälter mittels mindestens eines Durchflusssensors und Steuern, vorzugsweise über einen Regler (70), des ersten und/oder zweiten Gasregelventils anhand des vom mindestens einen Durchflusssensor gemessenen Gasflusses.

13. Verfahren nach einem der Ansprüche 10 bis 12, umfassend den zusätzlichen Schritt des Versorgens eines Verneblers (80), vorzugsweise über einen Druckregler und/oder über ein Vernebler-Gasregelventil, mit Gas aus dem Druckbehälter (40).

14. Medizinische Vorrichtung zum Versorgen eines Patienten mit einem Gas über einen Gasmischer nach einem der Ansprüche 1 bis 9, die weiterhin einen Analysator zur Analyse und/oder Anzeige von Beatmungsparametern eines Patienten umfasst, insbesondere eines Atemflusswertes eines Patienten, eines maximalen Atemvolumens eines Patienten, eines Atemminutenvolumens eines Patienten und/oder einer Atemfrequenz eines Patienten, anhand des Öffnungszustandes des Gasregelventils und/oder anhand eines Gasflusses, der von mindestens einem zur Messung des Flusses, vorzugsweise des Massenflusses, des zugeführten Gases angeordneten Durchflusssensor (16, 26) gemessen wurde.

**Revendications**

1. Mélangeur de gaz pour mélanger au moins deux gaz différents, le mélangeur de gaz comprenant

   - une première vanne de régulation de gaz (10) pour fournir un premier gaz,
   - une seconde vanne de régulation de gaz (20) pour fournir un second gaz,
   - un récipient sous pression (40) en communication fluidique avec les première et seconde vannes de régulation de gaz (10, 20) pour recevoir et stocker les premier et second gaz au-dessus de la pression atmosphérique, et
   - une vanne de régulation de gaz de sortie (50) en communication fluidique avec une sortie du récipient sous pression pour fournir le gaz mélangé provenant du récipient sous pression.

2. Mélangeur de gaz selon la revendication 1, dans lequel la vanne de régulation de gaz de sortie est une vanne de demande (50), de préférence une vanne de demande à basculement, détectant la demande en gaz d'un patient et fournissant le gaz mélangé en conséquence.

3. Mélangeur de gaz selon la revendication 1 ou 2, dans lequel le volume du récipient sous pression (40) se situe dans la plage de 0,01 l à 10 l, de préférence de 0,1 l à 3 l, de manière davantage préférée de 0,2 l à 0,5 l, de manière préférée entre toutes de 0,4 l.

4. Mélangeur de gaz selon l'une quelconque des revendications précédentes, dans lequel le débit maximal de la première et/ou seconde vanne de régulation de gaz est réglé entre 5 l/min et 350 l/min, de préférence entre 10 l/min et 200 l/min, de manière davantage préférée entre 140 l/min et 160 l/min, de manière préférée entre toutes à 150 l/min.

5. Mélangeur de gaz selon l'une quelconque des revendications précédentes, comprenant un dispositif de commande (70) connecté aux première et seconde vannes de régulation de gaz (10, 20) pour commander le fonctionnement des première et seconde vannes de régulation de gaz (10, 20) sur la base d'un rapport de mélange prédéterminé.

6. Mélangeur de gaz selon l'une quelconque des revendications précédentes, comprenant un capteur de pression (72) pour détecter la pression dans le récipient sous pression (40), le capteur de pression étant connecté, de préférence via un dispositif de commande (70), aux première et seconde vannes de régulation de gaz pour commander la fourniture des premier et second gaz dans le récipient sous pression.

7. Mélangeur de gaz selon l'une quelconque des revendications précédentes, incluant au moins un capteur de débit (16, 26) agencé pour détecter le débit, de préférence le débit massique, du premier et/ou du second gaz dans le récipient sous pression, le capteur de débit étant connecté, de préférence via un dispositif de commande (70), aux première et seconde vannes de régulation de gaz pour commander les première et/ou seconde vannes de régulation de gaz sur la base du débit de gaz mesuré par l'au moins un capteur de débit.

8. Mélangeur de gaz selon l'une quelconque des revendications précédentes, dans lequel la première et/ou la seconde vanne de régulation de gaz est une vanne de régulation de gaz électrique, hydraulique ou pneumatique pouvant être commandée par un dispositif de commande (70) et/ou dans lequel la première et/ou seconde vanne de régulation de gaz est un dispositif de commande du débit massique (100, 200).

9. Mélangeur de gaz selon l'une quelconque des revendications précédentes, dans lequel un nébuliseur (80) est en communication fluidique avec le récipient sous pression (40), de préférence via un régulateur de pression et/ou via une vanne de régulation de gaz de nébuliseur (82).

10. Procédé pour mélanger au moins deux gaz, comprenant les étapes de :

    - fourniture d'un premier gaz via une première vanne de régulation de gaz (10) dans un récipient sous pression (40),
    - fourniture d'un second gaz via une seconde vanne de régulation de gaz (20) dans le récipient sous pression (40),

- commande du débit des deux gaz par commande des première et seconde vannes de régulation de gaz pour obtenir un rapport de mélange prédéterminé des gaz dans le récipient sous pression (40),

- fourniture du gaz mélangé via une vanne de régulation de gaz de sortie (50) située en communication fluidique avec le récipient sous pression (40).

11. Procédé selon la revendication 10, comprenant les étapes de détection de la pression dans le récipient sous pression (40), d'ouverture des première et seconde vannes de régulation de gaz (10, 20) pour fournir du gaz dans le récipient sous pression (40) si la pression à l'intérieur du récipient sous pression est inférieure à un seuil inférieur prédéterminé, et de fermeture des première et seconde vannes de régulation de gaz (10, 20) si la pression à l'intérieur du récipient sous pression est supérieure à un seuil supérieur, la différence entre le seuil supérieur et le seuil inférieur étant de préférence dans une plage entre 0,01 et 2 bar, de manière davantage préférée entre 0,2 et 1,5 bar, de manière préférée entre toutes entre 0,5 et 1,4 bar.

12. Procédé selon la revendication 10 ou 11, comprenant les étapes additionnelles de détection du débit, de préférence du débit massique, du premier et/ou du second gaz dans le récipient sous pression au moyen d'au moins un capteur de débit, et de commande, de préférence via un dispositif de commande (70), des première et/ou seconde vannes de régulation de gaz sur la base du débit de gaz détecté par l'au moins un capteur de débit.

13. Procédé selon l'une quelconque des revendications 10 à 12, comprenant l'étape additionnelle de fourniture à un nébuliseur (80) du gaz provenant du récipient sous pression (40), de préférence via un régulateur de pression et/ou via une vanne de régulation de gaz de nébuliseur.

14. Dispositif médical pour fournir un gaz à un patient via un mélangeur de gaz selon l'une quelconque des revendications 1 à 9, incluant en outre un analyseur pour analyser et/ou afficher les paramètres respiratoires d'un patient, en particulier une valeur de débit respiratoire d'un patient, un volume respiratoire maximal d'un patient, un volume respiratoire par minute d'un patient et/ou une fréquence respiratoire d'un patient sur la base du statut d'ouverture de la vanne de régulation de gaz et/ou sur la base d'un débit de gaz mesuré par au moins un capteur de débit (16, 26) agencé pour détecter le débit, de préférence le débit massique, du gaz fourni.

Fig. 1

Fig. 2

Fig. 3

Fig. 4

Fig. 5

EP 2 489 392 B1

EP 2 489 392 B1

Fig. 6

22

$$(IFR)_1 \approx \frac{V}{p_1}\left(\frac{dp}{dt}\right)_1$$

$(IFR)_1$

dosing stop

dosing stop

UTH

$P_1$

$\frac{dt}{dp}$

$\left(\frac{dp}{dt}\right)$

LTH

dosing start

dosing start

$t_1$

CPU

P

GCV$_1$

V

DV

IFR

GCV$_2$

## Fig. 7

**EP 2 489 392 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007064986 A2

- US 4072148 A